# EUROPEAN PATENT APPLICATION

(11) **EP 4 785 911 A1**
(43) Date of publication of application: **05.08.2026**
(21) Application number: 25162014.2
(22) Date of filing: 06.03.2025
(51) Int. Cl.: A61F 2/30, A61F 2/32, A61F 2/44

(54) **ANCHORING ELEMENT FOR A JOINT IMPLANT, JOINT IMPLANT AND METHOD OF MANUFACTURING THE SAME**

(30) Priority: 29.01.2025 US 202563750884 P
(71) Applicant: ETH Zurich, 8092 Zurich (CH); Massachusetts Institute of Technology, Cambridge, MA 02139 (US); The Brigham and Women's Hospital Inc., Boston, MA 02115 (US)
(72) Inventor: Du, Xiaoyu, 8092 Zurich (CH); Ferguson, Stephen John, Zurich, 8092 (US); Traverso, Giovanni, Cambridge, MA 02139 (US)
(74) Representative: Longchamp, Jean-Nicolas

(57) **Abstract**

The present invention relates to anchoring element for a joint implant, a joint implant and a method of fabrication thereof. Specifically, the anchoring element for a joint implant according to the present invention comprises a bone-facing surface designed for the attachment of the joint implant to a bone and a hydrogel-facing surface designed for the attachment to a hydrogel component, wherein at least part of the hydrogel-facing surface is designed as a porous triply periodic minimal surface. The bonding between the hydrogel and the anchoring element is achieved through chemical anchoring and mechanical hooking mechanisms.

## Description

### Technical Field

This invention relates to the field of joint implants. More specifically, the present invention describes an anchoring element for a joint implant specifically designed for attachment to both a bone and a hydrogel component. The anchoring element is characterized by a porous triply periodic minimal surface on the side facing the hydrogel. The bonding between the hydrogel and the anchoring element is achieved through chemical anchoring and mechanical hooking mechanisms. The present invention relates also to a joint implant comprising an anchoring element according to the present invention as well as to a method for fabricating a corresponding joint implant.

### Background of the invention

Joint implants are medical devices that are used to replace damaged or diseased joints in the human body. These implants are typically used in orthopaedic surgeries to treat conditions such as osteoarthritis, rheumatoid arthritis, and other degenerative joint diseases. Joint implants can also be used in veterinary medicine to treat similar conditions in animals.

Joint implants are designed with several components to replicate the natural function of joints while ensuring long-term durability. The articulating surfaces, commonly made of materials such as metal, ceramic, or high-density polyethylene, facilitate smooth movement and reduce friction. Fixation components anchor the implant to the bone, utilizing bone cement or porous coatings like hydroxyapatite for cementless fixation, which encourages natural bone growth.

The revision rate, which corresponds to the percentage of implants requiring replacement or modification over time, serves as an important indicator of implant durability and long-term success. For example, hip and knee replacements typically show revision rates of around 5-10% and 6-12%, respectively, within 10-20 years. Factors influencing these rates include the implant design, material property, surgical technique, and patient-specific characteristics such as age, activity level, and overall health. Common causes for revision include implant loosening, wear, infection, or dislocation.

Recent advancements in materials and technology aim to reduce these revision rates and improve implant longevity. In particular, joint implants with a hydrogel cover have gained considerable attention due to their low coefficient of friction, which closely mimics the natural lubrication of cartilage. This property reduces wear and tear on the implant, enhances its performance, and promotes better joint mobility while minimizing post-surgical complications. In the current state of the art, the process of attaching a hydrogel to a titanium plate in joint implants often involves the use of additional clamps and glue. This method is not only complex and time-consuming, but it also introduces additional materials that may not be compatible with the body's biological environment. Furthermore, the mechanical properties of the titanium plate are often not well-matched to those of the bone, leading to issues such as stress shielding.

Innovations are thus necessary to develop simpler and more efficient techniques that accurately replicate the bone-cartilage interface, advancing joint implant technology.

### Summary of the invention

Thus, the object of the present invention is to propose a novel anchoring element for joint implants and a novel joint implant that eliminate or at least greatly reduces the above drawbacks.

According to the present invention, these objects are achieved in particular through the elements of the independent claims. Advantageous embodiments follow moreover from the dependent claims and the description.

In particular, the objects of the present invention are, in a first aspect, achieved by an anchoring element for a joint implant comprising a bone-facing surface designed for the attachment of the joint implant to a bone and a hydrogel-facing surface designed for the attachment to a hydrogel component, wherein at least part of the hydrogel-facing surface is designed as a porous triply periodic minimal surface.

Thanks to this anchoring element, it is possible to simplify the fabrication process of a joint implant, reduce material usage, and reach superior performance outcomes by featuring a unique mechanical hook between the anchor element and the hydrogel, eliminating the need for additional clamps and cement. The anchoring element comprises a bone-facing surface and a hydrogel-facing surface. The bone-facing surface is designed for the attachment of the joint implant to a bone, providing a secure and stable connection between the implant and the bone. This is crucial for the functionality and longevity of the joint implant, as it ensures that the implant remains in the correct position and can effectively perform its intended function.

The hydrogel-facing surface of the anchoring element is designed for attachment to a hydrogel component. Hydrogels are used in joint implants due to their unique properties, such as their ability to mimic the mechanical properties of natural tissues, their low coefficients of friction, their biocompatibility, and their capacity to absorb and retain large amounts of water. The attachment of the hydrogel component to the anchoring element is crucial for the overall performance of the joint implant, as it ensures that the hydrogel component remains securely in place and can effectively contribute to the function of the implant.

The unique feature of the anchoring element according to the present invention is that at least part of the hydrogel-facing surface is designed as a porous triply periodic minimal surface (TPMS). A TPMS is a continuous, smooth surface that minimizes its area locally while spanning a three-dimensional space in a periodic manner along three independent spatial directions. These surfaces exhibit translational symmetry in three directions, forming a repeating pattern, and are characterized by having zero mean curvature at every point. This unique structure provides a large surface area for the attachment of the hydrogel component, which can enhance the stability and durability of the attachment. Furthermore, the porosity of the TPMS allows for the infiltration of the hydrogel component into the anchoring element, which enhances the strength and stability of the attachment.

The use of a TPMS for the hydrogel-facing surface of the anchoring element provides several advantages. Firstly, it provides a large surface area for the attachment of the hydrogel component, allowing for a greater amount of hydrogel to bond with the anchoring element through chemical bonds, hydrogen bonds, and additional forces such as van der Waals forces and electrostatic interactions at the hydrogel-anchoring element interface. Secondly, the porosity of the TPMS allows for the infiltration of the hydrogel component into the anchoring element, which further enhances the strength and stability of the attachment. This also allows for the hydrogel component to be more effectively integrated with the anchoring element, which improves the overall performance of the joint implant. Thirdly, the TPMS structure transitions gradually without abrupt edges or points, which helps avoid areas of concentrated mechanical stress. This design feature minimizes the likelihood of stress accumulation at the interface between the hydrogel and the base material, leading to better mechanical compatibility and potentially enhancing durability of the joint implant. Furthermore, the TPMS structure's design avoids overhanging elements, which are typically challenging to print in additive manufacturing without support structures. This makes it highly compatible with layer-by-layer 3D printing techniques, enabling the production of customized and complex shapes, such as personalized joint implants. Additionally, the absence of overhangs and the optimized design minimize the need for support materials, reducing material waste and improving manufacturing efficiency.

In a first preferred embodiment of the first aspect of the present invention, the anchoring element is a metallic part. The use of a metallic part as the anchoring element provides several advantages. Firstly, metallic materials are known for their high strength and durability, which enhance the overall strength and longevity of the joint implant. This is particularly important for joint implants, which are subjected to significant mechanical stresses during use. Secondly, at least some metallic materials are biocompatible, which means that they are not harmful or toxic to the surrounding biological tissues. This is crucial for joint implants, which are implanted into the body and are in direct contact with biological tissues. Thirdly, metallic materials can be easily additive manufactured, which allows for the creation of the unique porous triply periodic minimal surface on the hydrogel-facing surface of the anchoring element. This enhances the attachment between the hydrogel component and the anchoring element, improving the overall performance of the joint implant.

Advantageously, the anchoring element is made from titanium alloys. Titanium alloys are particularly suitable for use in joint implants due to their unique properties. They are known for their high strength and low density, which makes them ideal for applications where strength-to-weight ratio is important. This is particularly relevant for joint implants, which need to be strong enough to withstand the mechanical stresses of the body, but also light enough to not add unnecessary weight. In addition to their strength and lightness, titanium alloys are also highly biocompatible. This means that they do not cause adverse reactions when implanted into the body, making them safe for use in medical applications. Furthermore, titanium alloys are resistant to corrosion, which enhances the longevity of the implant. The use of titanium alloys for the anchoring element also allows for the creation of the porous triply periodic minimal surface on the hydrogel-facing surface. Titanium alloys can be easily processed and additive manufactured, which facilitates the creation of this unique surface structure. This enhances the attachment between the hydrogel component and the anchoring element, improving the overall performance of the joint implant.

In a second preferred embodiment of the first aspect of the present invention, the anchoring element is a bioceramic part. Many bioceramic materials are biocompatible and exhibit excellent integration with surrounding biological tissues, minimizing the risk of adverse reactions and promoting natural tissue healing. Advantageously, the anchoring element is made from silicon nitride. The use of silicon nitride as the anchoring element offers several advantages. Firstly, silicon nitride is known for its exceptional mechanical properties, including high strength, durability, and wear resistance, which enhance the overall performance and longevity of joint implants subjected to significant mechanical stresses. Secondly, silicon nitride has demonstrated the ability to promote bone formation and integration with surrounding tissues, making it an excellent material for orthopedic applications. This characteristic supports better osseointegration and long-term stability of the implant. Thirdly, silicon nitride possesses inherent antibacterial properties, reducing the risk of infection and promoting a safer healing environment for the implant site. Finally, silicon nitride is compatible with advanced 3D printing techniques, such as stereolithography (SLA), which enables the creation of complex, customized geometries and unique porous structures, such as the TPMS surface on the hydrogel-facing surface of the anchoring element.

Both titanium and silicon nitride can advantageously be modified to have a hydrophilic surface through plasma treatment or treatment with a high-concentration alkaline solution. Following this, the surface of either titanium or silicon nitride can be grafted with specific functional groups using a silane solution to chemically anchor a specific hydrogel. For example, silane 3-(trimethoxysilyl)propyl methacrylate can be used to modify the surface of titanium or silicon nitride, providing a methacrylate terminal group that can copolymerize with the acrylate groups in acrylamide to anchor a polyacrylamide (PAAm) hydrogel. Surface modification of the anchoring element provides several advantages. Firstly, it introduces bonding groups on the surface, enhancing the adhesion between the hydrogel component and the anchoring element. This improves the stability and longevity of the joint implant, ensuring that the hydrogel component remains securely in place and contributes effectively to the function of the implant. Additionally, the hydrogel bonds to the surface of the anchoring element, which helps prevent detachment or washing off during the crosslinking process, maintaining the integrity of the hydrogel layer and ensuring consistent performance and functionality. Secondly, surface modification is a straightforward and cost-effective process that can be easily integrated into the manufacturing of the joint implant, making it a practical solution for enhancing the bond between the hydrogel component and the anchoring element.

In another preferred embodiment of the first aspect of the present invention, the bone-facing surface of the anchoring element features bone-fixing structures for securing the implant in place. The bone-fixing structures are designed to secure the implant in place, providing a stable and secure connection between the implant and the bone. This is crucial for the functionality and longevity of the joint implant, as it ensures that the implant remains in the correct position and can effectively perform its intended function. The bone-fixing structures can take various forms, depending on the specific requirements of the implant. For example, they could be protrusions or ridges that extend from the bone-facing surface of the anchoring element. These structures can be designed to interlock with the bone, providing a secure and stable connection. Alternatively, the bone-fixing structures could be openings or holes in the bone-facing surface of the anchoring element. These openings or holes can be designed to receive bone screws or other fixation devices, which can be used to secure the implant to the bone.

The use of bone-fixing structures on the bone-facing surface of the anchoring element provides several advantages. Firstly, it enhances the stability and security of the implant, ensuring that it remains in the correct position. Secondly, it can improve the longevity of the implant, as a secure and stable connection can reduce the risk of implant failure. Thirdly, it improves the functionality of the implant, as a secure and stable connection can ensure that the implant can effectively perform its intended function.

The anchoring element features bone-fixing structures advantageously in the form of anchoring teeth. These anchoring teeth are designed to engage with corresponding grooves on the bone surface, providing a secure and stable connection between the implant and the bone. This interlocking design enhances the stability of the implant, ensuring that it remains in the correct position and can effectively perform its intended function. The use of anchoring teeth as bone-fixing structures provides several advantages. Firstly, the interlocking design can provide a strong and secure connection between the implant and the bone, which enhances the stability and longevity of the implant. Secondly, the anchoring teeth can be designed to match the specific anatomy of the patient, ensuring a precise and secure fit. This improves the comfort and functionality of the implant, as it can be tailored to the specific needs of the patient. Thirdly, the anchoring teeth can be easily manufactured and incorporated into the design of the anchoring element, making them a practical and effective solution for securing the implant in place.

The anchoring teeth are advantageously designed to engage with corresponding grooves on the bone surface. This provides a secure and stable connection between the implant and the bone, enhancing the stability and longevity of the implant. Furthermore, this design allows for a greater surface area of contact between the implant and the bone, which can improve the strength and stability of the connection. This also allows for a more even distribution of forces across the implant, which reduces the risk of implant failure.

In a further preferred embodiment, the anchoring element features bone-fixing structures advantageously in the form of a TPMS stem, i.e. a stem having at least part of its surface as a TPMS surface. These TPMS stems are designed to facilitate bone growth into their open, interconnected structure, ensuring strong integration with the surrounding bone. The use of TPMS as bone-fixing structures provides several advantages. Firstly, the 3D connected open design allows for natural bone tissue ingrowth, which enhances the stability and long-term integration of the implant. Secondly, the porosity of the TPMS structure can be adjusted to match the mechanical properties of the surrounding bone, optimizing the load distribution and promoting better compatibility. Thirdly, the TPMS structure can be shaped into a stem and produced as a single, unified part through additive manufacturing, simplifying the fabrication process and reducing the need for multiple production steps.

In a second aspect, the present invention relates to a joint implant comprising an anchoring element according to the present invention and a hydrogel component attached to the hydrogel-facing surface of the anchoring element.

In a joint implant, the hydrogel component is a key part, providing cushioning and lubrication to mimic the natural function of cartilage in a joint. Hydrogels are biocompatible and have unique properties that make them suitable for use in joint implants. They can absorb and retain large amounts of water, which allows them to maintain their shape and elasticity under pressure. This makes them ideal for use in joints, which are subjected to significant mechanical stress. The attachment of the hydrogel component to the hydrogel-facing surface of the anchoring element is crucial for the overall performance of the joint implant. The unique design of the hydrogel-facing surface, with its porous triply periodic minimal surface, enhances the attachment between the hydrogel component and the anchoring element. This improves the stability and durability of the joint implant, ensuring that the hydrogel component remains securely in place and can effectively contribute to the function of the implant.

The joint implant, according to the present invention, provides several advantages. Firstly, the unique design of the anchoring element enhances the attachment between the hydrogel component and the anchoring element, improving the overall performance and longevity of the joint implant. Secondly, the use of a hydrogel component provides cushioning and lubrication to the joint, mimicking the natural function of cartilage. This improves the comfort and functionality of joint implants, enhancing the quality of life for the patient. Thirdly, the joint implant can be easily manufactured and assembled, making it a practical and effective solution for joint replacement surgery.

In a first preferred embodiment of the second aspect of the present invention, the hydrogel is chemically bonded, advantageously covalently bonded, to the hydrogel-facing surface of the anchoring element. The chemical bonding provides a strong and durable connection between the hydrogel component and the anchoring element, enhancing the stability and longevity of the joint implant. This is advantageous for the overall performance of the joint implant, as it ensures that the hydrogel component remains securely in place and can effectively contribute to the function of the implant.

The chemical bonding between the hydrogel and the hydrogel-facing surface of the anchoring element can be achieved through various methods. One advantageous method is through covalent bonding. Covalent bonds are strong and durable, making them ideal for securing the hydrogel component to the anchoring element. This enhances the stability and durability of the joint implant, ensuring that the hydrogel component remains securely in place even under the significant mechanical stresses that are experienced during use. The double anchor system (mechanical and chemical) between the anchoring element and hydrogel closely replicates the resilient bone-cartilage interface, enhancing the interfacial strength and improving the longevity and performance of the implant.

Advantageously, the hydrogel component comprises functional groups on its surface for covalent bonding with the anchoring element. These functional groups are designed for covalent bonding with the at least partially salinized surface of the anchoring element. Covalent bonds are strong and durable, making them ideal for securing the hydrogel component to the anchoring element. This enhances the efficiency and effectiveness of the chemical bonding process, improving the overall performance of the joint implant.

In another preferred embodiment of the second aspect of the present invention, the hydrogel component comprises a mixture of polyacrylamide (PAAm) and polyvinyl alcohol (PVA). PAAm is a polymer that is known for its high-water absorption capacity. This makes it ideal for use in hydrogels, as it can absorb and retain large amounts of water, allowing the hydrogel to maintain its shape and elasticity under pressure. This is advantageous for the functionality of the joint implant, as it ensures that the hydrogel component can effectively mimic the mechanical properties of natural cartilage. PVA, on the other hand, is a polymer that is known for its high mechanical strength and biocompatibility. This makes it ideal for use in joint implants, as it can withstand the significant mechanical stresses that are experienced during use, and it is not harmful or toxic to the surrounding biological tissues.

The use of a mixture of PAAm and PVA for the hydrogel component provides several advantages. Firstly, it combines the high-water absorption capacity of polyacrylamide with the high mechanical strength and biocompatibility of polyvinyl alcohol, enhancing the overall performance of the hydrogel component. Secondly, it allows for the hydrogel component to be tailored to the specific needs of the patient, as the properties of the hydrogel can be adjusted by varying the ratio of polyacrylamide to polyvinyl alcohol. Thirdly, both polyacrylamide and polyvinyl alcohol are readily available and easy to process, making them a practical and cost-effective choice for the production of hydrogels.

In yet another preferred embodiment of the second aspect of the present invention, the hydrogel is at least partially enclosed in a sheath of polyvinyl alcohol. The use of a sheath of PVA provides several advantages. Firstly, it protects the hydrogel component from mechanical damage, enhancing its durability and longevity. This is particularly important for joint implants, which are subjected to significant mechanical stresses during use. Secondly, it provides a barrier between the hydrogel component and the surrounding biological tissues, reducing the risk of adverse reactions. This is advantageous for joint implants, which are implanted into the body and are in direct contact with biological tissues. Thirdly, the sheath of polyvinyl alcohol enhances the stability of the hydrogel component, ensuring that it remains securely in place and can effectively contribute to the function of the implant.

The sheath of PVA can be easily manufactured and incorporated into the design of the joint implant, making it a practical and effective solution for protecting the hydrogel component. The sheath can be formed by various methods, such as by coating the hydrogel component with a solution of polyvinyl alcohol and then allowing it to be crosslinked via freeze-thaw and heat-annealing methods. The thickness of the sheath can be adjusted by varying the concentration of the polyvinyl alcohol solution and the size of the casting mold, allowing for the properties of the sheath to be tailored to the specific needs of the implant.

Advantageously, the sheath of polyvinyl alcohol comprises at least one active pharmaceutical ingredient, such as a steroid, an antibiotic and/or a growth factor. The inclusion of an active pharmaceutical ingredient in the sheath provides several advantages. Firstly, it allows for the localized delivery of the active pharmaceutical ingredient directly to the site of the implant. This enhances the effectiveness of the active pharmaceutical ingredient, as it can be delivered directly to the area where it is needed most. Secondly, it reduces the risk of systemic side effects, as the active pharmaceutical ingredient is delivered locally rather than systemically. This improves the safety and tolerability of the active pharmaceutical ingredient.

The active pharmaceutical ingredient can be incorporated into the sheath in various ways. For example, it can be mixed with the PVA solution before the sheath is formed. Alternatively, it can be incorporated into the sheath after it has been formed, for example by soaking the sheath in a solution containing the active pharmaceutical ingredient.

The active pharmaceutical ingredient can be any substance or combination of substances that have a therapeutic, pharmacological, or prophylactic effect. This could include, for example, anti-inflammatory drugs to reduce inflammation, analgesics to relieve pain, antibiotics to prevent or treat infection, or growth factors to promote healing. The specific active pharmaceutical ingredient or combination of active pharmaceutical ingredients used can be selected based on the specific needs of the patient and the intended use of the joint implant.

In another preferred embodiment of the second aspect of the present invention, the joint implant features a ball-and-socket structure, with one of the anchoring elements having a hydrogel-facing surface shaped into a cup and covered with hydrogel. The other anchoring element has a hydrogel-facing surface shaped into a semi-ball structure, also covered with hydrogel. This design is particularly suitable for hip and shoulder implants due to their joint shapes. The use of hydrogel-on-hydrogel articulating surfaces provides several advantages. Firstly, the ultra-low friction between the hydrogel surfaces reduces wear rates, enhancing the longevity of the implant and minimizing potential wear-related complications. Secondly, the ball-and-socket configuration allows for a range of motion that mimics natural joint movement, improving the functionality and comfort for the patient.

In a further preferred embodiment of the second aspect of the present invention, the joint implant comprises two anchoring elements, wherein the hydrogel-facing surfaces of the anchoring elements are facing each other, and the hydrogel component is located between the two hydrogel-facing surfaces. The use of two anchoring elements provides several advantages. Firstly, it provides a secure and stable environment for the hydrogel component, ensuring that it remains securely in place and can effectively contribute to the function of the implant. Secondly, the use of two anchoring elements allows the joint implant to be used in situations where the implant needs to be placed between two bones, such as a spinal implant. Thirdly, the use of two anchoring elements with the hydrogel enables the joint implant to provide six degrees of freedom due to the flexibility of the hydrogel core, which is particularly important for use of the implant in the spinal system. The two anchoring elements can be identical or different, depending on the specific requirements of the spinal implant. They can be designed to match the specific anatomy of the patient, ensuring a precise and secure fit. This can improve the comfort and functionality of the spinal implant, enhancing the quality of life for the patient.

In a further preferred embodiment of the second aspect of the present invention, the joint implant comprises two anchoring elements each having a hydrogel component bounded to its hydrogel-facing surface and wherein a further hydrogel component is provided between the hydrogel components bounded to the anchoring elements. The additional hydrogel component is advantageously either partially tethered or freely mobile between hydrogel-coated anchoring elements above and below, with the additional hydrogel component optionally extending beyond the articulating surfaces of the joint. This design mimics the semi-independent behavior of natural structures like the menisci in the knee or the labrum in the hip or shoulder, providing cushioning, load distribution, and stability. The tethered hydrogel allows controlled movement under load, while the freely mobile version dynamically self-adjusts within a constrained space. Extensions of the hydrogel beyond the articulation emulate peripheral soft tissues, enhancing congruence, shock absorption, and joint sealing and stability. The hydrogel's size, shape, and mechanical properties can be tailored to replicate specific anatomical features, offering ultra-low friction, improved wear resistance, and biomechanical compatibility to enhance implant durability and patient comfort. This embodiment is particularly beneficial for complex joints such as the knee, hip, and shoulder, where the integration of soft, adaptive hydrogel structures significantly improves functionality and longevity.

The joint implant can be designed for use in either veterinary or human applications. This flexibility in application broadens the potential use of the joint implant, making it a versatile solution for a range of different joint replacement needs.

In veterinary applications, the joint implant can be used in a variety of different animals, including but not limited to dogs, cats, horses, and livestock. The specific design of the joint implant, including the size and shape of the anchoring elements and the hydrogel component, can be tailored to the specific anatomy and needs of the animal. This improves the comfort and functionality of the implant, enhancing the quality of life for the animal.

In human applications, the joint implant can be used in a variety of different joints, including but not limited to the knee, hip, shoulder, spine, ankle and elbow. The specific design of the joint implant, including the size and shape of the anchoring elements and the hydrogel component, can be tailored to the specific anatomy and needs of the patient. This improves the comfort and functionality of the implant, enhancing the quality of life for the patient.

Regardless of whether the joint implant is used in veterinary or human applications, the unique design of the anchoring element and the hydrogel component provide a range of benefits, including a reduced wear rate for increased durability, enhanced stability and longevity, improved comfort and functionality, and a lower risk of implant failure. In a third aspect, the present invention relates to a method for manufacturing a joint implant, comprising:
a. Providing an anchoring element according to the present invention;
b. Depositing a hydrogel component or a hydrogel precursor on the hydrogel-facing surface of the anchoring element; and
c. Binding the hydrogel component to the anchoring element.

The first step of the method involves providing an anchoring element. This anchoring element is designed with a bone-facing surface for attachment to a bone and a hydrogel-facing surface for attachment to a hydrogel component. At least part of the hydrogel-facing surface is designed as a porous triply periodic minimal surface, as previously described. Preferred manufacturing methods include additive manufacturing, casting, machining, forging. The second step of the method involves depositing a hydrogel component or a hydrogel precursor on the hydrogel-facing surface of the anchoring element. The hydrogel precursor can be prepared as a liquid solution or sol using pre-gel components, preferably water-soluble monomers. If a chemical crosslinking mechanism is used, a crosslinking agent is added to create strong bonds between polymer chains, enhancing the stability and properties of the hydrogel. Additionally, a double-network pre-gel solution can be mixed to improve the hydrogel's strength, toughness, and water-absorbing capabilities. The third step of the method involves binding the hydrogel component on the anchoring element. Binding the hydrogel component to the anchoring element can be achieved through various methods, depending on the specific material of the anchoring element and the hydrogel component. For example, the hydrogel component can be chemically bonded to the anchoring element, for example through covalent bonding.

The method for manufacturing a joint implant according to the present invention provides several advantages. Firstly, it allows for the production of joint implants with a unique design for different joints, enhancing the performance and longevity of the implants. Secondly, it is a straightforward and efficient method, making it a practical and cost-effective solution for the production of joint implants. Thirdly, it allows for the production of joint implants that can be tailored to the specific needs of the patient, improving the comfort and functionality of the implants.

### Brief description of the drawings

- Figure 1 shows a first preferred embodiment of an anchoring element and of a joint implant according to the present invention;
- Figure 2 shows experimental results of biocompatibility tests;
- Figure 3 shows experimental results of drug release tests;
- Figure 4 demonstrates the antibacterial efficacy of antibiotic loaded implants according to the present invention;
- Figure 5 shows a second preferred embodiment of an anchoring element and of a total hip replacement joint implant according to the present invention;
- Figure 6 shows experimental results of load tests of hip implants according to the present invention;
- Figure 7 shows a third preferred embodiment of an anchoring element and of a joint implant according to the present invention;
- Figure 8 illustrates the six degrees of freedom of an artificial intervertebral disc;
- Figure 9 shows experimental results of load tests of artificial disc implants according to the present invention;
- Figure 10 presents experimental results of tensile tests;
- Figure 11 shows a flow diagram of a method for manufacturing a joint implant according to a preferred embodiment of the present invention; and
- Figure 12 shows manufactured prototypes of various joint implant parts according to the present invention.

### Detailed description of preferred embodiments and presentation of experimental results

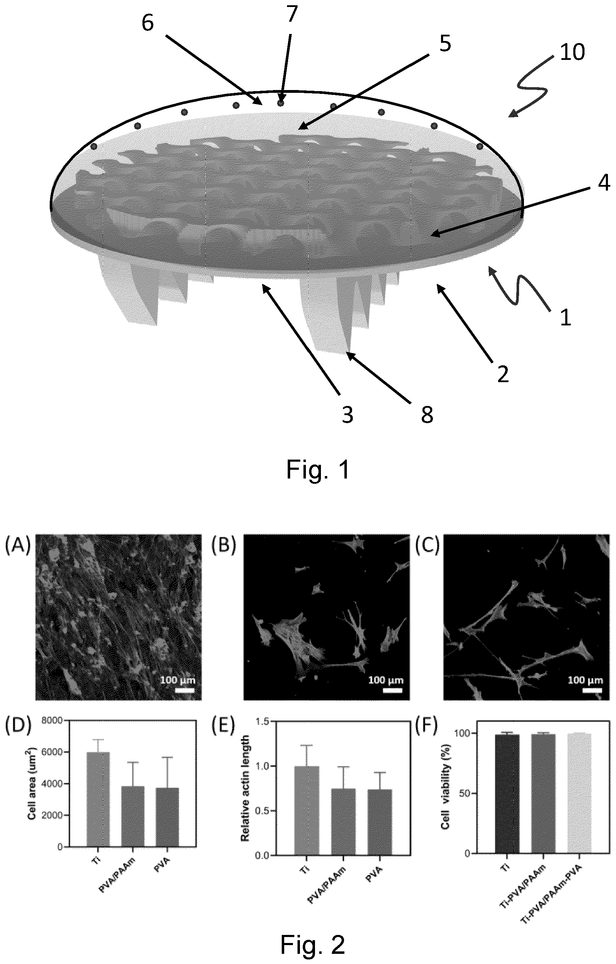

Figure 1 illustrates an anchoring element 1 and a joint implant 10, here a resurfacing implant, according to a first preferred embodiment of the present invention. In the embodiment of Figure 1, the anchoring element 1 is made of a titanium part 2 comprising a bone-facing surface 3 designed for the attachment of the joint implant to a bone and a hydrogel-facing surface 4 designed for the attachment to a hydrogel component 5. According to the present invention, the hydrogel-facing surface 4 is designed as a porous triply periodic minimal surface for facilitating and improving the binding of the hydrogel component to the anchoring element. Notably, the shape and porosity of the titanium part can be adjusted to approach the mechanical properties of bones in different joints, thus avoiding stress shielding. Owing to rapid developments in 3D printing technology, the titanium part with various designs can be easily and efficiently fabricated. In order to secure the implant 10 to the bone, the anchoring element 1 comprises bone-fixing structures 8 for securing the implant in place. Furthermore, implant 10 comprises an enclosing PVA sheath 6. The enclosing PVA sheath not only enhances the mechanical properties of the implant but also has the potential to carry active pharmaceutical ingredients 7 as for example anti-infection drugs, thereby preventing infection after surgery.

Advantageously, the hydrogel 5 is a flexible yet resilient double-network hydrogel, combining physical crosslinks in Polyvinyl Alcohol (PVA) and chemical crosslinks in Polyacrylamide (PAAm). It demonstrates outstanding impact and fatigue resistance. The gel has a high-water content to replicate the pressurized interstitial fluid of cartilage, responsible for its load-bearing properties and low friction.

In order to test the load bearing and energy absorption abilities of resurfacing implants according to the first preferred embodiment of the present invention, double-network PVA/PAAm hydrogels with various mix ratios and crosslinking methods were prepared. The hydrogel comprises a covalently crosslinked PAAm network and a PVA network with crystalline structures. PAAm synthesis involved free-radical polymerization of acrylamide, using N,N-methylenebisacrylamide as a crosslinker, ammonium persulfate as a thermal initiator, and TEMED as an accelerator. PVA crystallinity was induced using freeze-thaw (FT) and heat-anneal (HA) methods.

The compressive strength of the hydrogel (HA group) surpasses 40 MPa in an unconfined condition, which is within the range of cartilage compressive strength. Impact tests using a drop tower showed that the HA group fully recovered from ~60% deformation, while the FT-treated hydrogel bounced back from ~80% deformation without damage within the hydrogel or at the gel/titanium interface. Both hydrogels exhibited excellent damping properties, absorbing significant impact energy, suggesting potential for protecting surrounding bone tissue from fractures. Additionally, the water content and stiffness could be adjusted to suit different joint areas.

Two mechanisms were used to connect the anchoring elements and hydrogel, mimicking the cartilage-bone interface. Chemical bonding of hydrogels to titanium was achieved through silane modification. Specifically, grafted silane 3-(trimethoxysilyl) propyl methacrylate (TMSPMA) has a methacrylate terminal group, enabling copolymerization with acrylate groups in acrylamide during free radical polymerization. To enhance bonding between the hydrogel and titanium plate, the hydrogel-facing surface of the titanium plate was designed as a triply periodic minimal surface (TPMS) structure, characterized by a mean curvature of zero at each point, providing varying surface properties without sharp edges. This porous TPMS structure facilitates gel penetration into the grooves, encapsulating the gel through interaction with the titanium plate. The enclosing PVA sheath 6 exhibited a measured coefficient of friction of approximately 0.03 under a ball-on-disc test using an alumina ball, which is significantly lower than the typical coefficient of friction for metal implants, ranging from 0.1 to 0.3.

To assess the biocompatibility and cellular activity of the hydrogel-metal implant system, cellular attachment and morphology of human bone marrow mesenchymal stem cells (hBMSCs) on different parts of the implants were examined, including titanium plate (Ti), PVA/PAAm, and PVA. This evaluation was performed through staining with phalloidin and DAPI solution. The results revealed that most of the cells in all groups exhibited a round and well-spread morphology on the implant surfaces (Figure 2 (A-C)). Notably, some differences were observed between the Ti group and the PVA/PAAm or PVA groups. The Ti group exhibited a higher number of adhered cells, which can be attributed to its stiffer and more textured surface compared to the other groups, facilitating enhanced cell adhesion. Additionally, cells in the PVA/PAAm or PVA groups displayed an elongated spindle-shaped morphology without thick stress fibers, while the Ti group exhibited distinct and thick stress fibers. Further analysis involved the quantification of actin length and cell area using confocal laser scanning microscopy (CLSM). Although no statistically significant difference was observed in these parameters among the groups, the Ti group showed a higher cell area and longer actin length (Figure 2 (D-E)), suggesting stronger focal adhesion in the Ti group. Additionally, the viability and cytotoxicity of the implants was studied. The results, as depicted in Figure 2 (F), indicated that the majority of cells in all groups were alive, with cell viability exceeding 95%. This finding demonstrates that all components of the implants are biocompatible and do not exhibit cytotoxicity from chemicals, confirming the absence of crosslinker residues from hydrogel synthesis.

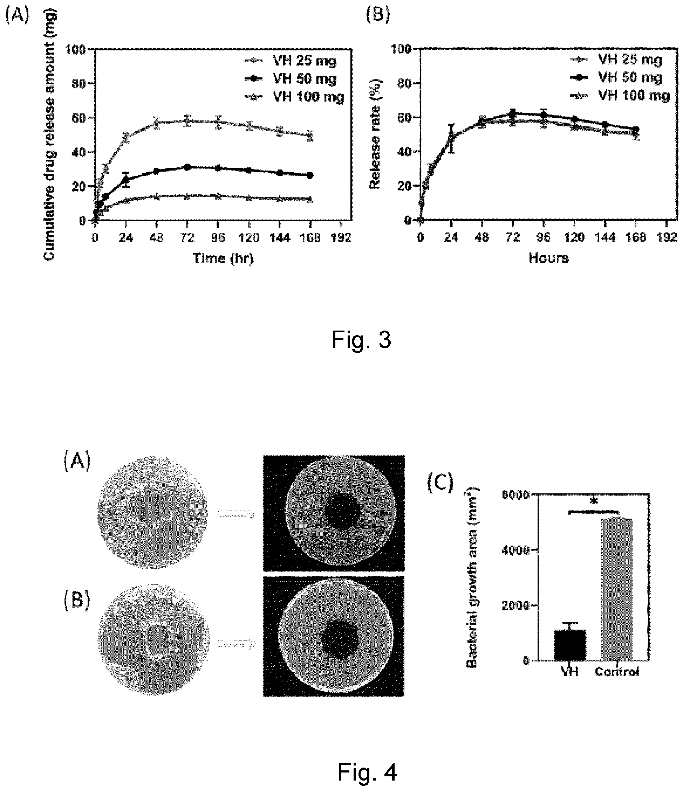

The PVA sheath in the present invention not only encloses the hydrogels to provide strong load-bearing properties but also has the advantageous additional function for drug loading and eluting. In order to demonstrate the antibacterial effect of an antibiotic loaded implant according to the present invention, vancomycin, an antibiotic used to treat various bacterial infections, including those caused by methicillin-resistant *Staphylococcus aureus.* The release kinetics of vancomycin from the hydrogel-metal implant was evaluated in PBS at pH 7.4 and 37°C using high-performance liquid chromatography (HPLC). An initial burst release occurred on the first day, followed by a gradual plateau. HPLC data indicated vancomycin degradation after 2-3 days. Increasing amounts of vancomycin (25 mg, 50 mg, and 100 mg per implant) were loaded into the sheath, with consistent cumulative release percentages across all concentrations (Figure 3 (A-B)). This suggests vancomycin does not react with the PVA sheath, and even the highest drug load remained below the percolation threshold of the PVA solution. The release kinetics fit an exponential plateau model for the first 48 hours. Different drug contents resulted in varying release amounts, allowing modulation of release kinetics based on therapeutic needs.

The antibacterial efficacy of the vancomycin-loaded implant against methicillin-sensitive *Staphylococcus aureus* (MSSA) was assessed using the agar diffusion method. Bacteria remained near the implant without drugs (Figure 4 (A)), while a significant reduction in bacterial viability was observed with the vancomycin-loaded implant (Figure 4 (B)). Quantifying the residual bacterial growth area revealed that the drug-loaded implants significantly inhibited MSSA growth by 78.25% ± 4.55% compared to the control (Figure 4 (C)). These promising results indicate that the drug-eluting implant could inhibit inflammation post-surgery.

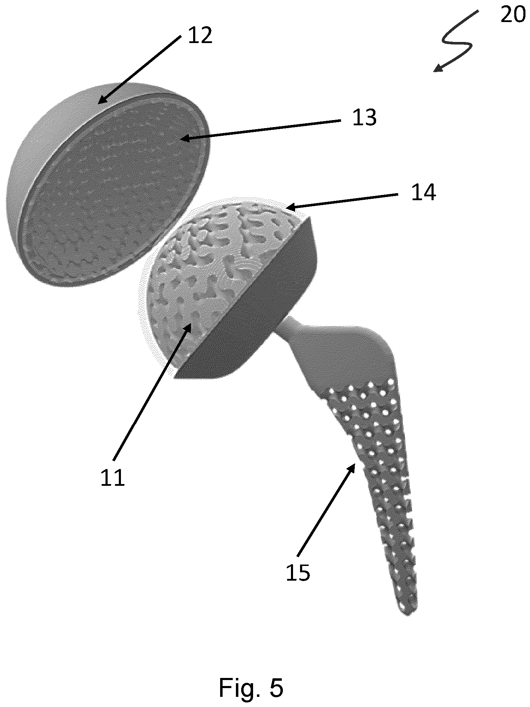

Figure 5 illustrates another preferred embodiment of a joint implant 20, here a hip replacement implant, according to the present invention. The total hip implant design was divided into five individual components to showcase their separate parts: the femoral head11, the acetabular cup 12, the acetabular cup hydrogel 13, the femoral head hydrogel 14 and the femoral stem 15. Two TPMS structure variations were advantageously incorporated into the hip implant components, one with a porosity of 40% and unit size of 5 mm for the femoral head and acetabular cup to mechanically hook the hydrogel, and another with a porosity of 40% and unit size of 7mm for the femoral stem to provide bigger pores for bone tissue integration. Note that the porosity of the TPMS structure can be easily adjusted to match the mechanical properties of the surrounding bone.

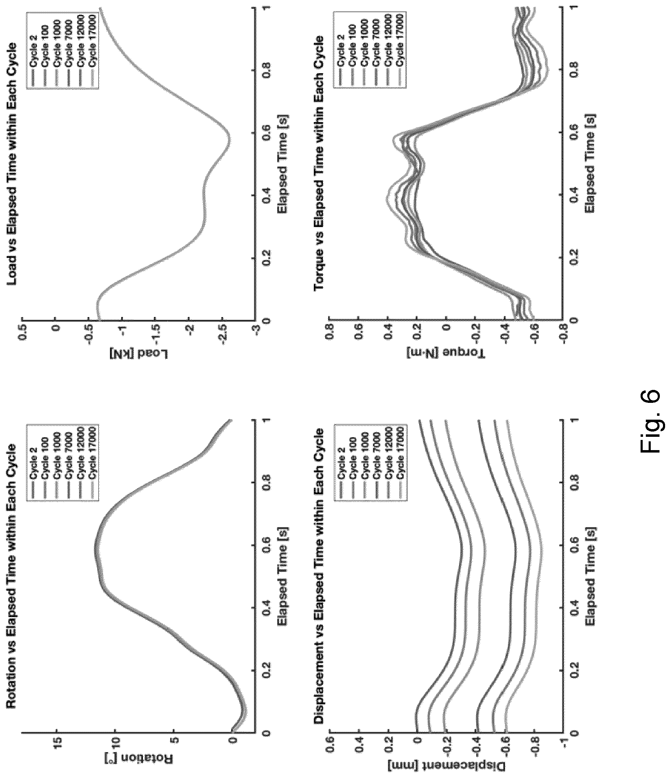

In order to test the mechanical properties of implants according to Figure 5, a titanium acetabular cup and femoral head with a clamping beam were designed and 3D printed, then covered with a hydrogel lining. These components were clamped into a dynamic material testing machine (Instron E10000, UK), with the acetabular cup and femoral head positioned to ensure the hydrogel linings were in contact. Rotational testing, adapted from ISO 14242 (Implants for surgery - Wear of total hip-joint prostheses), was performed to evaluate the hydrogel lining's performance. A time-varying force with a peak nominal load of 3000 N was applied to the femoral head at 1 Hz frequency, with a 12-degree rotational amplitude. The system underwent 18,000 cycles per day, resting in water overnight, and repeated for 7 consecutive days (126,000 cycles). During each daily test, the load and rotation remained consistent over 18,000 cycles. The displacement of the femoral head decreased slightly (~0.8 mm) to maintain the applied force. The forces on the hip joint can range from 1 to 5 times body weight, with higher forces during activities like running, jumping, and landing. The hip implant successfully sustained the 3000 N force during long-term cyclic loading while maintaining high lubrication. The torque increased slightly during the test, peaking at less than 0.8 Nm, corresponding to a coefficient of friction of less than 0.01, even in the absence of lubricant. In comparison, the coefficient of friction for metal-on-metal implants typically ranges from 0.20-0.27, metal-on-polyethylene from 0.06-0.08, and ceramic-on-ceramic from 0.002-0.07. The hydrogel-on-hydrogel contact in the implant according to the present invention showed much lower friction than most conventional materials. During a 5-hour friction test, the hydrogel lost a small percentage of water but regained its original water content overnight, mimicking the physiological rest period in human joints. Results (Figure 6) from each subsequent day exhibited a consistent trend, with the initial torque remaining the same as on the previous day. The combination of the extremely low COF and strong strength suggests that hydrogel-on-hydrogel contact in implant system of the present invention provides significantly less friction, potentially improving the performance and longevity of the implant by reducing wear and tear.

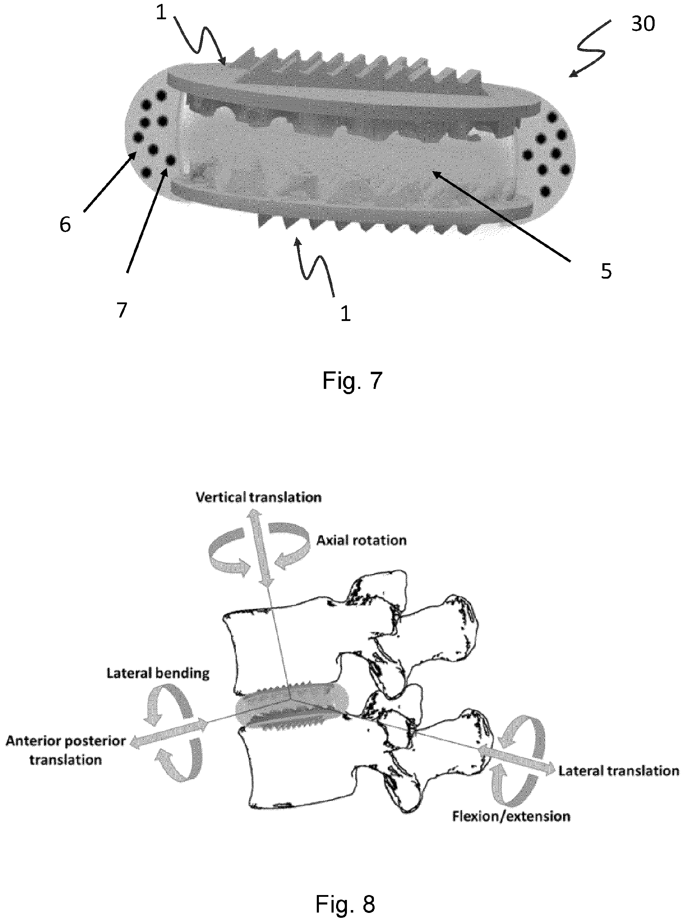

Figure 7 illustrates a further preferred embodiment of a joint implant according to the present invention. In this embodiment, the join implant 30 comprises two anchoring elements 1 sandwiching the hydrogel 5, and a sheath 6 with antibiotics 7 encloses the anchoring elements 1 and the hydrogel 5 This embodiment represents an implementation of the present invention as spinal implants, specifically as total disc replacement implants. The present inventions allow replacing traditional total disc replacement implants, which typically rely on a rigid ball-and-socket design, with a flexible hydrogel layer connecting two titanium endplates, allowing the implant to closely mimic the natural intervertebral disc by providing six degrees of freedom, similar to a natural disc (see Figure 8).

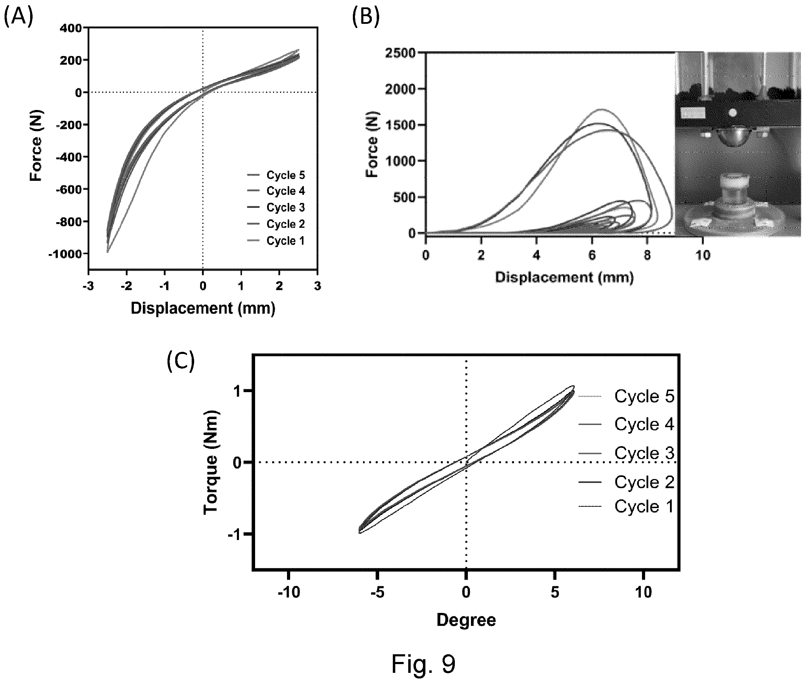

The core of the artificial disc, according to this preferred embodiment of the present invention, is a robust double-network hydrogel (PVA/PAAm) that is both flexible and resilient. Supported by an outer PVA sheath, the artificial disc demonstrated exceptional impact resistance, as tested using a drop tower setup (Figure 9(B)). With a diameter of 30 mm, it withstood a maximum impact force of 1546 ± 156 N and absorbed 2.70 ± 0.06 J of energy during an initial impact at a velocity of 1.59 ± 0.02 m/s and total energy of 3.18 ± 0.07 J. Notably, the disc absorbed over 80% of the impact energy, highlighting its significant energy-absorbing capability. Additionally, it can tolerate substantial loads of up to approximately 1000 N for a lumbar disc under cyclic loading (Figure 9(A)) and 1 Nm torque under rotational movement (Figure 9(C)). Importantly, the results from both the tensile-compressive and impact tests depict substantial hysteresis loops, a characteristic of robust damping properties. This attribute closely emulates natural intervertebral disc behavior, crucial for accurately replicating the dynamic traits inherent to the actual intervertebral disc. To assess the fatigue resistance of the artificial disc system, a cyclic test was conducted. The implants endured 10,000 cycles of compression and tension, retaining their capacity to restore their original shape without any signs of failure.

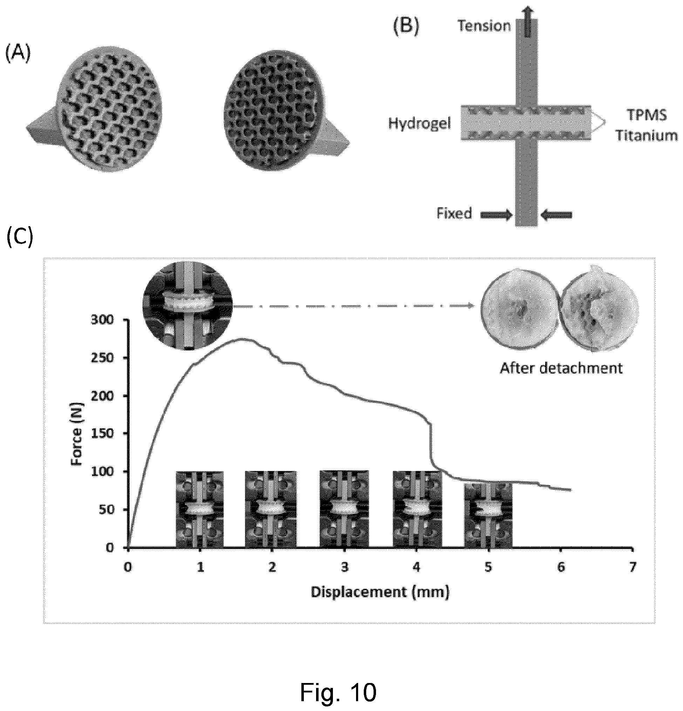

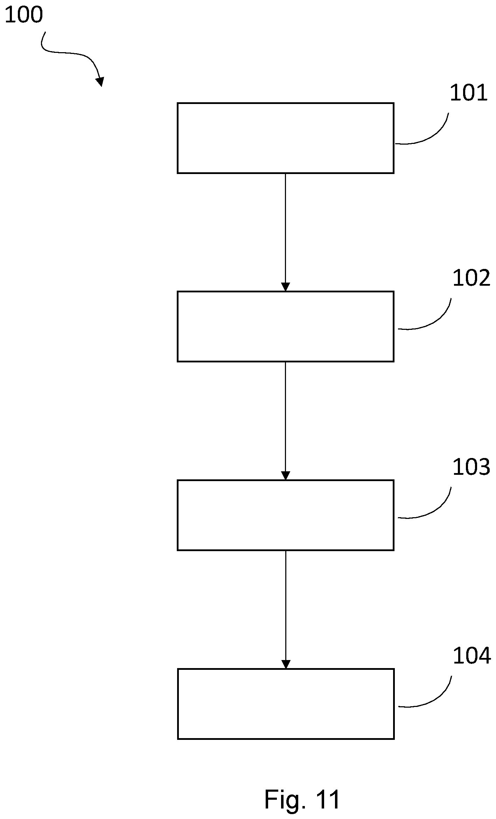

For further tests, the hydrogel component is positioned between titanium anchoring endplates. This TPMS design facilitates gel infiltration and adhesion, creating a stable integration between the hydrogel and endplates. Tensile tests on the TPMS titanium plates bonded with PVA/PAAm gels were conducted, as shown in Figure10(B). Panel (A) of Figure 10 shows 3D printed titanium anchoring plate with TPMS structure on its hydrogel-facing surface, panel (B) illustrates the tensile test demonstration setup, and panel (C) shows a force-displacement curve of the tensile test on the hydrogel-metal sample using a materials testing machine at a tensile testing rate of 1 mm/min. The results revealed that the elongation of the hydrogel under tensile force created pore-like spaces, ultimately leading to gel rupture. However, the hydrogel-metal interface remained intact, indicating that the TPMS structure effectively secures the gel to the titanium anchoring plate. Figure 11 shows a flow diagram of a preferred embodiment of the manufacturing method 100 according to an embodiment of the present invention. The first step 101 of the method involves providing an anchoring element. This anchoring element is designed with a bone-facing surface for attachment to a bone and a hydrogel-facing surface for attachment to a hydrogel component. At least part of the hydrogel-facing surface is designed as a porous triply periodic minimal surface, as previously described.

The second step 102 of the method involves preparing a liquid solution or sol using pre-gel components, preferably water-soluble monomers. Advantageously, a crosslinking agent is added to create strong bonds between polymer chains, enhancing the stability and properties of the hydrogel. Additionally, a double-network pre-gel solution can be mixed to improve the hydrogel's strength, toughness, and water-absorbing capabilities.

The third step 103 of the method involves depositing and binding a pre-gel component on the anchoring element. The hydrogel component can be deposited on the hydrogel-facing surface of the anchoring element in various ways. For example, the hydrogel component can be applied as a liquid or sol, which can then be solidified to form a solid hydrogel component. Binding the hydrogel component to the anchoring element can be achieved through various methods, depending on the specific design of the anchoring element and the hydrogel component. For example, the hydrogel component can be chemically bonded to the anchoring element, for example through covalent bonding.

The fourth step 104 of the method involves crosslinking treatment of the hydrogel, which can be achieved using various methods depending on the type of hydrogel component. For example, in the case of a double-network hydrogel, crosslinking can be performed through physical crosslinking using freeze-thaw cycles and heating-annealing process, as well as chemical crosslinking via free radical polymerization when a crosslinker is added to the pre-gel.

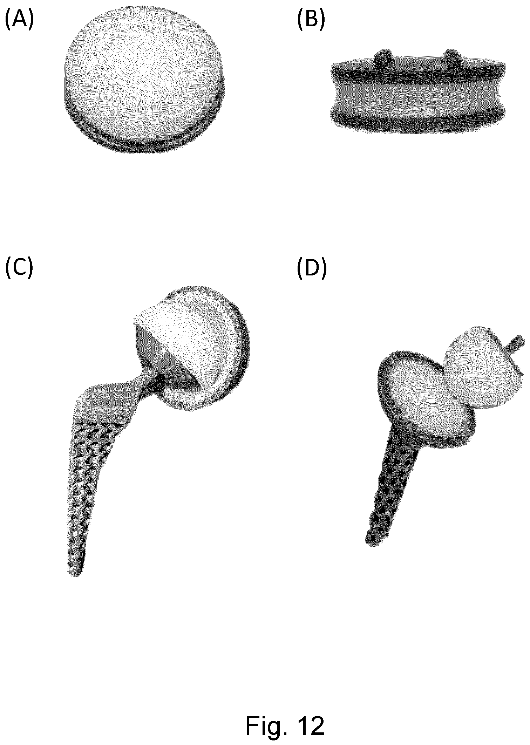

Figure 12 shows manufactured prototypes of various joint implant parts: (A) a resurfacing implant, (B) an artificial disc, (C) a total hip replacement implant, and (D) a shoulder implant.

## Claims

1. An anchoring element for a joint implant comprising a bone-facing surface designed for the attachment of the joint implant to a bone and a hydrogel-facing surface designed for the attachment to a hydrogel component,
**characterized in that**
at least part of the hydrogel-facing surface is designed as a porous triply periodic minimal surface (TPMS).

2. Anchoring element according to claim 1 wherein it is a metallic part, advantageously made from titanium alloys, or is made from a bioceramic, advantageously silicon nitride.

3. Anchoring element according to claims 1 or 2, wherein the bone-facing surface of the anchoring elements features bone-fixing structures for securing the implant in place.

4. Anchoring element according to claim 3, wherein the bone-fixing structures are in the form of anchoring teeth, wherein the anchoring teeth are advantageously designed to engage with corresponding grooves on the bone surface.

5. Anchoring element according to claim 3, wherein the bone-fixing structures are in the form of a TPMS stem, with the anchoring stem being advantageously designed to include pores that facilitate bone growth and provide mechanical properties that match those of the surrounding bone.

6. A joint implant for veterinary or human use comprising an anchoring element according to any one of the claims 1 to 5 and a hydrogel component attached to the hydrogel-facing surface of the anchoring element, wherein the hydrogel is advantageously chemically bonded, in particular covalently bonded, to the hydrogel-facing surface of the anchoring element.

7. Joint implant according to claim 6 wherein the hydrogel component comprises on its surface functional groups such as acrylate group for covalent bonding with the anchoring element, wherein at least part of the surface of the anchoring element is advantageously salinized with specific functional group such as methacrylate terminal group.

8. Joint implant according to any one of the claims 6 or 7, wherein the hydrogel component comprises a mixture of polyacrylamide (PAAm) and polyvinyl alcohol (PVA).

9. Joint implant according to any one of the claims 6 to 8, wherein the hydrogel is at least partially enclosed in a sheath of polyvinyl alcohol, and wherein the sheath of polyvinyl alcohol comprises advantageously at least one active pharmaceutical ingredient, such as a steroid, an antibiotic and/or a growth factor.

10. Joint implant according to any one of the claims 6 to 9, wherein it comprises two anchoring elements, and wherein one of the anchoring elements has a hydrogel-facing surface shaped into a cup and covered with hydrogel, while the other anchoring element has a hydrogel-facing surface shaped into a semi-ball structure, also covered with hydrogel, with the design being particularly suitable for hip and shoulder implants due to their joint shapes to provide hydrogel on hydrogel articulating surface.

11. Joint implant according to any one of the claims 6 to 10, wherein it comprises two anchoring elements and wherein the hydrogel-facing surfaces of the anchoring elements are facing each other, and the hydrogel component is located between the two hydrogel-facing surfaces.

12. Joint implant according to claim 11, wherein it comprises two anchoring elements each having a hydrogel component bounded to its hydrogel-facing surface and wherein a further hydrogel component is provided between the hydrogel components bounded to the anchoring elements.

13. A method for manufacturing a joint implant according to any one of claims 6 to 12, comprising:
a. Providing an anchoring element according to the present invention;
b. Depositing a hydrogel component or an hydrogel precursor on the hydrogel-facing surface of the anchoring element; and
c. Binding the hydrogel component to the anchoring element.

14. Method according to claim 13, wherein it comprises a step of saline modification of at least part of the anchoring element.

15. Use of a metal or bioceramic component comprising at least one surface designed as a porous triply periodic minimal surface as hydrogel anchoring element in a joint implant.
